# FASCICULE DE BREVET EUROPEEN

(11) **EP 0 540 388 B1**
(45) Date de publication et mention de la délivrance du brevet: **28.06.1995**
(21) Numéro de dépôt: 92402804.6
(22) Date de dépôt: 14.10.1992
(51) Int. Cl.: C07C 213/10

(54) **Procédé d'élimination de la diéthanolamine présente dans la triéthanolamine et procédé de préparation de triéthanolamine purifiée**
Verfahren zur Entfernung von in Triethanolamine anwesenden Diethanolamin und Verfahren zur Herstellung von reinen Triethanolamin
Process for the removal of the diethanolamine present in triethanolamine, and process for the preparation of pure triethanolamine

(30) Priorité: 29.10.1991 FR 9113329
(43) Date de publication de la demande: 05.05.1993
(73) Titulaire: SOCIETE FRANCAISE HOECHST, 92800 Puteaux (FR)
(72) Inventeur: Claude, Gabrielle, F-75018 Paris (FR); Blanc, Alain, F-93200 Saint-Denis (FR)
(74) Mandataire: Rinuy, Santarelli

(56) Documents cités:
- GB-A- 1 140 867

## Description

La présente invention concerne un procédé d'élimination de la diéthanolamine présente dans la triéthanolamine, son application et un procédé de préparation de triéthanolamine purifiée.

Aujourd'hui, on sait que la diéthanolamine, à cause de par ses propriétés chimiques vis à vis des agents nitrosants, présente, dans certains cas, des propriétés cancérigènes. C'est le cas notamment pour certaines préparations complexes contenant de la diéthanolamine destinés à des compositions phytosanitaires ou cosmétiques, ou a l'obtention d'huiles de coupe pour lesquelles les expérimentations sur l'animal ont démontré leur caractère cancérigène. En conséquence, il se pose le problème de l'élimination de la diéthanolamine, désignée aussi 2,2'-iminodiéthanol, des matières premières utilisées pour l'obtention de ces compositions et notamment l'élimination de la diéthanolamine présente dans la triéthanolamine, qui est largement employée dans les formulations d'huile de coupe.

La triéthanolamine obtenue industriellement par condensation de l'oxyde d'éthylène avec de l'ammoniac contient des quantités plus ou moins importantes de diéthanolamine. Les points d'ébullition de ces deux amines ne permettent pas leur séparation parfaite ; en conséquence même distillée la triéthanolamine contient encore souvent des quantités supérieures à 0,1 % en poids de diéthanolamine qu'il est recommandé d'éliminer pour certaines applications afin d'éviter les inconvénients cités précédemment.

Or la demanderesse a découvert un procédé simple et efficace pour réaliser cette élimination, et obtenir de la triéthanolamine purifiée de la diéthanolamine.

Le procédé selon la présente invention est caractérisé par le fait que l'on traite la triéthanolamine contenant de la diéthanolamine avec du glyoxal dans un rapport molaire glyoxal sur diéthanolamine supérieur ou égal à 1, puis que, si désiré, l'on isole la triéthanolamine du milieu réactionnel par des moyens connus en soi. La triéthanolamine à traiter peut être isolée, ou en mélange ou être incluse dans une composition.

Dans des conditions préférentielles de mise en oeuvre de l'invention, le procédé ci-dessus décrit est réalisé de la manière suivante :
- en atmosphère inerte,
- à température comprise entre 20 et 80 ° C,
- avec un rapport molaire glyoxal sur diéthanolamine compris entre 1 et 2,
- avec du glyoxal, soit en solution aqueuse de concentration supérieure à 40 % en poids, soit sous forme de trimère dihydrate solide,
- en suivant l'élimination de la diéthanolamine par prélèvement régulier dans le milieu réactionnel d'échantillons dans lesquels on recherche la diéthanolamine résiduelle, notamment par analyse chromatographique en phase gazeuse.

Le glyoxal est très réactif vis à vis de la diéthanolamine et il la transforme très rapidement et quantitativement en N,N-bis (hydroxy-2 éthyl) glycine. Si désiré, en fin de réaction on peut isoler la triéthanolamine du milieu réactionnel, notamment par distillation sous pression réduite. On obtient ainsi de la triéthanolamine pure.

Il est connu que la triéthanolamine s'oxyde lentement à l'air en se colorant et en libérant progressivement notamment de la diéthanolamine, du formaldéhyde et que la diéthanolamine libérée s'oxyde aussi lentement à l'air en donnant également des produits colorés. En conséquence, la triéthanolamine purifiée par le procédé de l'invention devra être conservée soit soigneusement à l'abri de l'oxygène (KIRK-OTHMER, Encyclopedia of Chemical Technology, 3 ème édition, volume 1, New York, John Wiley and Sons, 1978, pages 944-950), soit en présence d'un antioxydant ou de tout autre moyen connu inhibant les effets de l'oxygène.

Le procédé selon l'invention permet d'obtenir très facilement de la triéthanolamine contenant moins de 100 ppm en poids de diéthanolamine en partant de triéthanolamine pouvant contenir jusqu'à 2 % en poids (20 000 ppm) de diéthanolamine. En fin de traitement par le procédé selon l'invention, on peut, si désiré, obtenir de la triéthanolamine pure en soumettant par exemple le milieu réactionnel à une distillation sous pression réduite.

La présente invention a également pour objet l'utilisation du procédé ci-dessus décrit pour purifier des compositions renfermant de la triéthanolamine comportant de la diéthanolamine à titre d'impureté.

La présente demande a enfin pour objet un procédé de préparation de triéthanolamine substantiellement dépourvue de diéthanolamine et d'une composition renfermant de la triéthanolamine substantiellement dépourvue de diéthanolamine, caractérisé par le fait que l'on traite la triéthanolamine contenant la diéthanolamine ou la composition renfermant de la triéthanolamine contenant de la diéthanolamine avec du glyoxal dans un rapport molaire glyoxal sur diéthanolamine supérieur ou égal à 1, puis que l'on isole la triéthanolamine ou la composition renfermant la triéthanolamine du milieu réactionnel.

Les exemples suivants illustrent la présente demande sans toutefois la limiter. Dans ces exemples, la diéthanolamine présente dans la triéthanolamine a été dosée par chromatographie en phase gazeuse selon le procédé décrit ci-après.
- Pour doser la diéthanolamine présente dans la triéthanolamine par chromatographie en phase gazeuse, on la transforme en son dérivé triacétylé par réaction d'une quantité connue de triéthanolamine avec 100 volumes d'un mélange anhydride acétique-pyridine 20-80 en volume pesé exactement, pendant 30 minutes, à 80 °C en vase clos, puis après refroidissement à 20 °C, on dissout dans le milieu réactionnel 2 mg de triméthoxy-3,4,5 benzoate de méthyle qui servira d'étalon interne et enfin on injecte 1 »l de cette solution dans l'injecteur réglé à 250 °C d'un chromatographe en phase gazeuse équipé d'un détecteur à piègeage d'ions (ITD), et d'une colonne capillaire de 25 mètres et d'un diamètre de 0,25 mm dont le revêtement intérieur est un film d'épaisseur 0,25 »m de polydiméthylsiloxane, maintenue en isotherme à 1500 °C, avec un débit d'hélium de 1 ml/min. Auparavant, on avait déterminé le coefficient de réponse du dérivé triacétylé de la diéthanolamine par rapport au triméthoxy-3,4,5 benzoate de méthyle dans les mêmes conditions. Le pourcentage pondéral de diéthanolamine est calculé à partir des surfaces des pics de la diéthanolamine et de l'étalon, et corrigé par les poids des prises d'essai et du coefficient de réponse.

### EXEMPLE 1 :

On abandonne à la température ambiante, pendant 24 heures, un mélange de 149,2 g (1 mole) de triéthanolamine distillée contenant 0,35 % en poids de diéthanolamine, soit 0,52 g (5 mmoles) avec 1,45 g d'une solution aqueuse commerciale de glyoxal à 40 % en poids, soit 0,58 g (10 mmoles) de glyoxal. On obtient ainsi une solution de triéthanolamine contenant 0,007 % de diéthanolamine soit 0,10 mmole. Cette solution de triéthanolamine peut être utilisée telle quelle pour l'obtention de formulation d'huile de coupe. Si on le souhaite, une distillation sous une pression réduite de 0,3 mbar à 163 °C permet d'obtenir de la triéthanolamine pure avec un rendement quasi quantitatif.

### EXEMPLE 2 :

On abandonne à 80 °C, en atmosphère inerte, pendant 24 heures un mélange de 149,2 g (1 mole) de triéthanolamine distillée contenant 0,35 % en poids de diéthanolamine avec 0,73 g d'une solution aqueuse de glyoxal à 40 % en poids. On obtient ainsi après refroidissement du milieu réactionnel à la température ambiante, une solution de triéthanolamine contenant 0,01 % de diéthanolamine soit 0,14 mmole.

### EXEMPLE 3:

On abandonne à 20 °C, en atmosphère inerte, pendant 170 heures, un mélange de 149,2 g (1 mole) de triéthanolamine distillée contenant 0,35 % en poids de diéthanolamine avec 0,36 g d'une composition aqueuse de glyoxal à 80 % en poids soit 5 mmoles de glyoxal. On obtient ainsi de la triéthanolamine contenant 0,01 % de diéthanolamine.

### EXEMPLE 4 :

On abandonne à 80 °C, en atmosphère inerte pendant 24 heures, un mélange de 149,2 g (1 mole) de triéthanolamine distillée contenant 0,35 % en poids de diéthanolamine avec 0,55 g d'une composition aqueuse de glyoxal à 80 % en poids, soit 7,5 mmoles de glyoxal. On obtient ainsi de la triéthanolamine renfermant 0,004 % de diéthanolamine.

## Revendications

1. Procédé d'élimination de la diéthanolamine présente dans la triéthanolamine caractérisé par le fait que l'on traite la triéthanolamine contenant de la diéthanolamine avec du glyoxal dans un rapport molaire glyoxal sur diéthanolamine supérieur ou égal à 1.

2. Procédé selon la revendication 1, caractérisé par le fait que le rapport glyoxal sur diéthanolamine est compris entre 1 et 2.

3. Procédé selon l'une quelconque des revendications 1 et 2 caractérisé par le fait qu'il est réalisé à une température comprise entre 20 °C et 80 °C.

4. Procédé selon l'une quelconque des revendications 1 à 3 caractérisé par le fait que le glyoxal utilisé est en solution aqueuse de concentration pondérale supérieure à 40 %.

5. Procédé selon l'une quelconque des revendications 1 à 3, caractérisé par le fait que le glyoxal utilisé est à l'état de trimère dihydrate solide.

6. Utilisation du procédé selon l'une quelconque des revendications 1 à 5, pour éliminer la diéthanolamine de compositions renfermant de la triéthanolamine.

7. Procédé de préparation de triéthanolamine substantiellement dépourvue de diéthanolamine, caractérisé par le fait que l'on traite la triéthanolamine contenant la diéthanolamine avec du glyoxal dans un rapport molaire glyoxal sur diéthanolamine supérieur ou égal à 1, puis que l'on isole la triéthanolamine du milieu réactionnel.

8. Procédé de préparation d'une composition renfermant de la triéthanolamine substantiellement dépourvue de diéthanolamine, caractérisé par le fait que l'on traite la composition renfermant de la triéthanolamine contenant la diéthanolamine avec du glyoxal dans un rapport molaire glyoxal sur diéthanolamine supérieur ou égal à 1, puis que l'on isole la composition du milieu réactionnel.

## Claims

1. Process for the elimination of the diethanolamine present in triethanolamine wherein the triethanolamine containing diethanolamine is treated with glyoxal in a molar ratio of glyoxal to diethanolamine greater than or equal to 1.

2. Process according to claim 1, wherein the ratio of glyoxal to diethanolamine is comprised between 1 and 2.

3. Process according to any one of claims 1 and 2 wherein it is carried out at a temperature comprised between 20°C and 80°C.

4. Process according to any one of claims 1 to 3 wherein the glyoxal used is in aqueous solution of a concentration greater than 40% by weight.

5. Process according to any one of claims 1 to 3 wherein the glyoxal used is in the solid dihydrate trimer state.

6. Use of the process according to any one of claims 1 to 5, for the elimination of diethanolamine from compositions containing triethanolamine.

7. Preparation process for triethanolamine substantially free from diethanolamine, wherein the triethanolamine containing the diethanolamine is treated with glyoxal in a molar ratio of glyoxal to diethanolamine greater than or equal to 1, then the triethanolamine is isolated from the reaction medium.

8. Preparation process for a composition containing triethanolamine substantially free from diethanolamine, wherein the composition containing the triethanolamine which contains diethanolamine is treated with glyoxal in a molar ratio of glyoxal to diethanolamine greater than or equal to 1, then the composition is isolated from the reaction medium.

## Patentansprüche

1. Verfahren zur Entfernung von in Triethanolamin vorliegendem Diethanolamin, dadurch gekennzeichnet, daß man das Diethanolamin enthaltende Triethanolamin mit Glyoxal in einem molaren Verhältnis von Glyoxal zu Diethanolamin von größer als oder gleich 1 behandelt.

2. Verfahren nach Anspruch 1, dadurch gekennzeichnet, daß das Verhältnis von Glyoxal zu Diethanolamin zwischen 1 und 2 liegt.

3. Verfahren nach einem der Ansprüche 1 und 2, dadurch gekennzeichnet, daß es bei einer Temperatur zwischen 20 und 80°C durchgeführt wird.

4. Verfahren nach einem der Ansprüche 1 bis 3, dadurch gekennzeichnet, daß das verwendete Glyoxal in Form einer wäßrigen Lösung einer Gewichtskonzentration von größer als 40 % vorliegt.

5. Verfahren nach einem der Ansprüche 1 bis 3, dadurch gekennzeichnet, daß das verwendete Glyoxal in Form des festen trimeren Dihydrates vorliegt.

6. Anwendung des Verfahrens nach einem der Ansprüche 1 bis 5 zum Entfernen von Diethanolamin aus Triethanolamin umfassenden Zusammensetzungen.

7. Verfahren zur Herstellung von Triethanolamin, das von Diethanolamin im wesentlichen befreit ist, dadurch gekennzeichnet, daß man das Diethanolamin enthaltende Triethanolamin mit Glyoxal in einem molaren Verhältnis von Glyoxal zu Diethanolamin von größer als oder gleich 1 behandelt, worauf man das Triethanolamin aus dem Reaktionsmedium isoliert.

8. Verfahren zur Herstellung einer Zusammensetzung, die Triethanolamin umfaßt, das von Diethanolamin im wesentlichen befreit ist, dadurch gekennzeichnet, daß man die Zusammensetzung, die das diethanolaminhaltige Triethanolamin umfaßt, mit Glyoxal in einem molaren Verhältnis von Glyoxal zu Diethanolamin von größer als oder gleich 1 behandelt, worauf man die Zusammensetzung aus dem Reaktionsmedium isoliert.
